# EUROPEAN PATENT APPLICATION

(11) **EP 4 528 273 A1**
(43) Date of publication of application: **26.03.2025**
(21) Application number: 22869438.6
(22) Date of filing: 16.09.2022
(51) Int. Cl.: G01N 33/50, G01N 1/28, G01N 27/26

(54) **QUALITY CONTROL SOLUTION AND USE THEREOF**

(30) Priority: 18.09.2021 CN 202111098611
(71) Applicant: Leadway (HK) Limited, Sheung Wan, Hong Kong (CN)
(72) Inventor: YE, Jing, Hangzhou, Zhejiang 310030 (CN); SUN, Yulong, Hangzhou, Zhejiang 310030 (CN); ZHANG, Li, Hangzhou, Zhejiang 310030 (CN)
(74) Representative: Schiweck Weinzierl Koch Patentanwälte Partnerschaft mbB
(86) International application number: PCT/CN2022/119452
(87) International publication number: WO 2023/041072

(57) **Abstract**

The present invention provides a quality control solution and its use. Said quality control solution includes a buffer, a polymer, EDTA or a salt thereof, a preservative, a blood simulated dye and an analyte, said polymer being selected from PEG 2000~ 6000, propylene glycol, glycerol, or a combination thereof. The quality control solution of the present invention can be for performing quality control of at least one analyte. It does not require refrigeration, freezing or lyophilization, can be placed at room temperature, is relatively convenient to use and can be used in fields of immunological detection, biochemical detection and electrochemical detection.

## Description

### Field of the Invention

The present invention mainly relates to a quality control solution, in particular to a quality control solution suitable for immunological detection, biochemical detection and electrochemical detection.

### Background of the Invention

Discussion of issues such as the early prevention and treatment of chronic kidney disease (CKD) and the delay of its progression has become a common concern in public health and clinical medicine. In recent years, the focus of kidney disease community has shifted from chronic renal failure and replacement therapy to the early stage of CKD. Clinical investigations have shown that early intervention treatment on patients with CKD can significantly reduce the mortality of patients, effectively delay the progression of the condition, reduce or even avoid the occurrence of end-stage kidney disease, and also help to reduce medical costs of patients. Therefore, a multifunctional analyzer with the function of detecting three indicators (blood creatinine, blood urea and blood uric acid) of the renal function has market demand and space. As a kidney disease detecting instrument, it can effectively detect the illness degree of kidney disease of a patient by detecting the contents of creatinine, urea and uric acid in the blood of the patient, and make a corresponding determination, which can be used as a basis for selecting further therapeutic regimens.

When a test strip is used to detect in an electrochemical manner at least one of creatinine, urea and uric acid in a biological fluid such as blood and urine, it requires to use creatinine, urea and/or uric acid with known concentration(s) for performing quality control. During testing, the test strip is inserted into a test strip insertion hole in an electrochemical test instrument, a quality control solution is added to the sample addition port of the test strip, and then whether the reading of the electrochemical test instrument is in the normal quality control range is observed. If it is in the normal quality control range, it indicates that the electrochemical test instrument and the test strip are normal, and if it is out of the normal quality control range, the electrochemical test instrument or the test strip should be replaced.

However, in the prior art, quality control solutions used for electrochemical biological detection are mostly single-item quality control solutions, and there's few quality control solutions for two or more items, which have disadvantages of poor stability, liability of influencing other items and liability of resulting in inaccurate detection results. At present, most of the quality control solutions in the market require refrigeration, freezing or lyophilization, and are not convenient to use.

### Summary of the Invention

In view of the deficiencies of the prior art, the present invention provides a quality control solution, which comprises a buffer, a polymer, EDTA or a salt thereof, a preservative, a blood simulated dye and an analyte, and is applicable to fields of immunological detection, biochemical detection and electrochemical detection.

The present invention further provides a composite quality control solution, comprising a buffer, a polymer, EDTA or a salt thereof, a preservative, a blood simulated dye, and at least two of the three analytes: urea, sodium urate and creatinine. The present invention further provides a quality control solution for renal function detection, comprising a buffer, a polymer, EDTA or a salt thereof, a preservative, a blood simulated dye, and at least one of the three analytes: urea, sodium urate and creatinine. The present invention further provides a quality control solution for detection of creatinine, comprising a buffer, a polymer, EDTA or a salt thereof, a preservative, a blood simulated dye, creatinine and creatine.

Further, the buffer has a concentration of 0.01 mol/L to 2 mol/L and a pH of 6.5 to 7.5, the concentration of the polymer is 10% to 40% (w/w), the concentration of EDTA or the salt thereof is 0.1% to 1% (w/w), the concentration of the preservative is 0.1% to 2% (w/w), and the concentration of the blood simulated dye is 0.1% to 2% (w/w).

Further, said analyte is selected from urea, sodium urate, creatinine or a combination thereof. Further, when said analyte comprises creatinine, the concentration of the creatinine is 0.0001% to 3% (w/w); when said analyte comprises sodium urate, the concentration of the sodium urate is 0.0001% to 3% (w/w); and when said analyte comprises urea, the concentration of the urea is 0.0001% to 3% (w/w). Further, when said analyte comprises creatinine, said quality control solution further comprises creatine. Further, the creatine has a concentration of 0.0001% to 3% (w/w).

Further, said polymer is selected from PEG 2000~6000, propylene glycol, glycerol or a combination thereof. Further, said blood simulated dye is selected from Sulforhodamine B, Congo Red, Mito Red, Acid Red, Fluorescent Red, Allura Red, Cresol Red, Amaranth, Chlorophenol Red, and Sulforhodamine 101. Further, said preservative may be selected from sodium azide, thimerosal, sodium benzoate, Proclin-150, Proclin-200, Proclin-300 or Proclin-5000.

Further, said quality control solution further comprises calcium chloride or sodium chloride, or a combination of calcium chloride and sodium chloride. Further, when said quality control solution comprises calcium chloride, the calcium chloride has a concentration of 0.02% to 0.5% (w/w); and when said quality control solution comprises sodium chloride, the sodium chloride has a concentration of 0 to 0.1% (w/w). Further, said buffer is a Good's buffer. Preferably, said Good's buffer is selected from HEPES (4-(2-hydroxyethyl)piperazine-1-ethanesulfonic acid), MES (2-morpholinoethanesulphonic acid), BES (N,N-bis(2-hydroxyethyl)-2-aminoethanesulfonic acid) and TAPSO (3-[N-tris-(hydroxymethyl)methylamino]-2-hydroxypropanesulphonic acid). Most preferably, the Good's buffer is selected from TAPSO. Further, said Good's buffer has a concentration of 0.05 mol/L to 0.5 mol/L and a pH of 6.5 to 7.2.

The present invention also refers to use of said quality control solution in detection of urea, creatinine and urea.

The present invention has the following beneficial effects: (1) The present invention does not simply add several analytes (creatinine, urea and uric acid) to a two-item quality control solution or a three-item quality control solution: first, uric acid is unstable in an aqueous solution, and as sodium urate is relatively more stable, sodium urate is selected to replace uric acid; second, creatinine is unstable in an aqueous solution, addition of a certain amount of creatine can prevent obvious changes of the creatinine concentration, and third, in addition to selecting a suitable Good's buffer, it is also necessary to find a suitable pH value to keep a plurality of components in the two-item or three-item quality control solutions relatively stable (for example, sodium urate is poor in stability and its concentration will decrease significantly when the alkalinity of the two-item or three-item quality control solution is strong), and a certain electrical signal scan be maintained when an electrochemical test strip is used for testing. (2) The two-item or three-item quality control solution of the present invention can be used for detecting creatinine, urea and uric acid in an electrochemical manner, and can perform quality control for at least two of the three analytes at the same time without separately preparing a quality control solution for each analyte. (3) The quality control solution of the present invention doesn't require refrigeration, freezing or lyophilization, can be placed at room temperature, and is more convenient to use.

### Brief Description of the Drawings

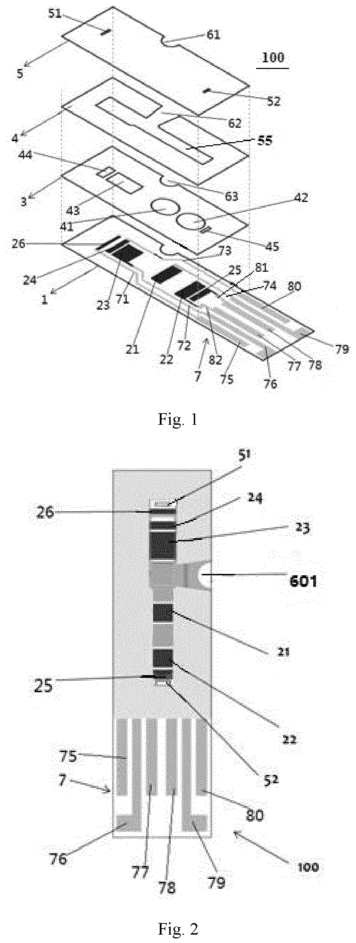

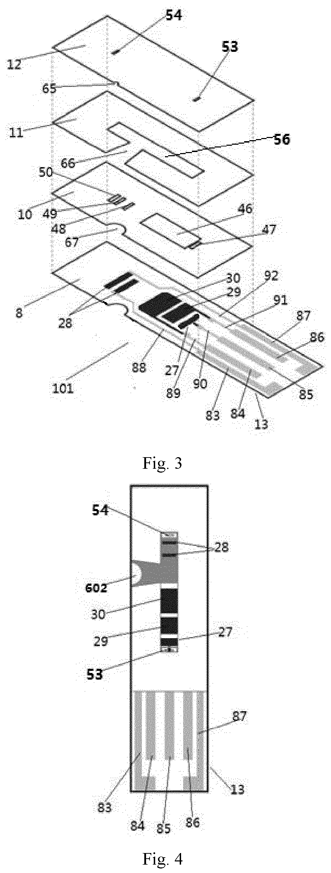

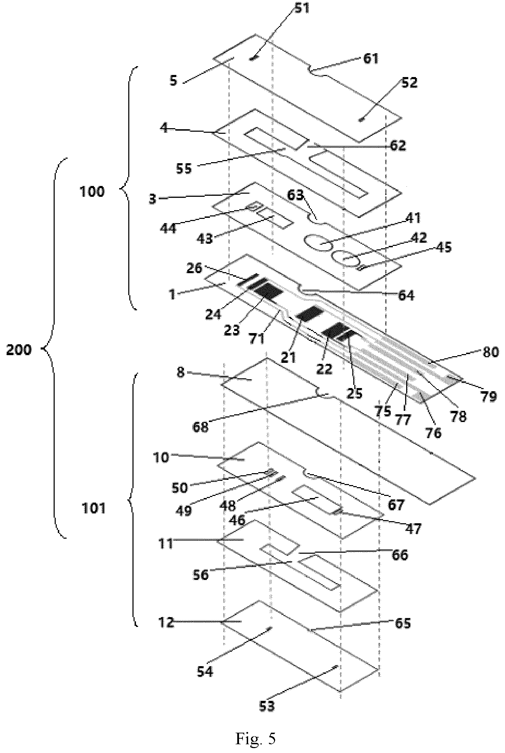

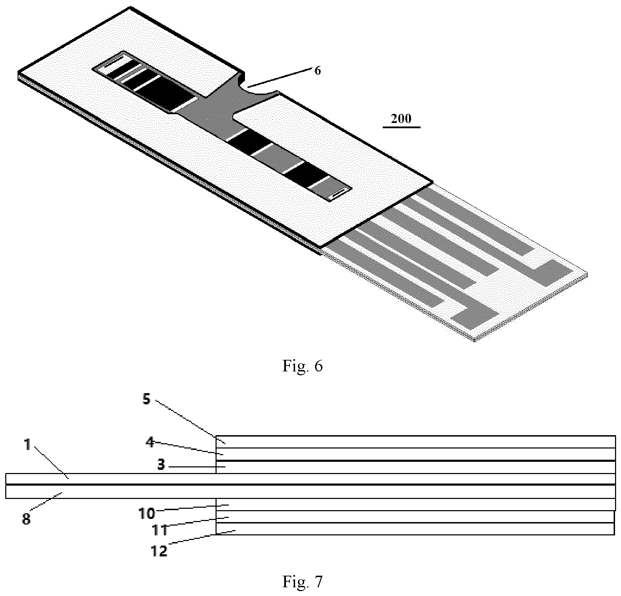

Fig. 1 is an exploded view of a first biosensor of a first electrochemical test strip. Fig. 2 is a schematic diagram of the first biosensor in Fig. 1 when being assembled together. Fig. 3 is an exploded view of a second biosensor of the first electrochemical test strip. Fig. 4 is a schematic diagram of the second biosensor in Fig. 3 when being assembled together. Fig. 5 is an exploded view of a second electrochemical test strip. Fig. 6 is a schematic diagram of the electrochemical test strip in Fig. 5 when being assembled together. Fig. 7 is a lateral schematic diagram of the electrochemical test strip, with the first biosensor and the second biosensor not sharing the same insulating substrate.

### Detailed Description of the Embodiments

The following examples further illustrate the present invention. These examples are not intended to limit the scope of the present invention, but provide further understanding of the present invention.

The quality control solution of the present invention includes a Good's buffer, a polymer, EDTA or a salt thereof, a preservative, a blood simulated dye and an analyte, said polymer being selected from PEG 2000~6000, propylene glycol, glycerol, or a combination thereof. Said analyte is selected from urea, sodium urate, creatinine or a combination thereof, which means that said analyte may be either one of urea, sodium urate and creatinine, or a combination of any two of urea, sodium urate and creatinine, or a combination of the three (urea, sodium urate and creatinine) themselves. Said blood simulated dye can cause the quality control solution of the present invention to be red, and is used for simulating the color of blood to facilitate observing the sample addition situation.

In the present invention, PEG 2000 ~ 6000 refers to polyethylene glycol with a molecular weight: 2000 to 6000. Preferably, the quality control solution of the present invention further contains calcium chloride, which plays roles of electric conduction and a stabilizer. EDTA or the salt thereof plays the role of a stabilizer in the quality control solution of the present invention. EDTA or the salt thereof is preferably selected from EDTA, disodium EDTA or dipotassium EDTA. Preferably, a suitable amount of sodium chloride can also be added to the quality control solution of the present invention, which can serve the function of enhancing the ion strength, increase test current values of uric acid and creatinine and improve electrical signal gradients.

The two-item quality control solution or three-item quality control solution of the present invention is not prepared by simple combination of several analytes. In view of the instability and poor solubility of uric acid in conventional methods, the quality control solution of the present invention uses sodium urate to replace uric acid when being used for performing quality control of uric acid. In addition, sodium urate has relatively poor stability and obviously reduced concentration when the pH value of the quality control solution is more alkaline, therefore, in addition to selecting suitable buffer and polymer, it is necessary to find a suitable pH value so that various components in the quality control solution, including sodium urate, remain relatively stable during storage and maintain certain electrical signal when an electrochemical test strip is used for testing. When the quality control solution of the present invention contains creatinine, creatinine is unstable in an aqueous solution, and preferably, the addition of a certain amount of creatine can prevent significant changes of the concentration of creatinine.

The quality control solution of the present invention is usually used in conjunction with a test strip in the fields of immunity, biochemistry and electrochemistry. A test strip in the electrochemical field (hereinafter referred to as an electrochemical test strip) is taken as an example here for detailed description. As shown in Figs. 1 to 7, said electrochemical test strip 200 includes a first biosensor 100 and a second biosensor 101, and the first biosensor 100 and the second biosensor 101 do not share the same insulating substrate. The first biosensor 100 has a first electrical connection end 7 and the second biosensor 101 has a second electrical connection end 13. The first electrical connection end 7 and the second electrical connection end 13 are located at the same end of the electrochemical test strip 200, and can be connected with an electrical connector of a detecting instrument. The sample addition port of said electrochemical test strip 200 includes a first sample addition port 601 located on the lateral side of the first biosensor 100 and a second sample addition port 602 located on the lateral side of the second biosensor 101.

The first biosensor 100 is sequentially provided with a first insulating substrate 1, a first electrode system disposed on the first insulating substrate 1, a first reaction region forming layer 3, a first channel forming layer 4 and a first upper cover layer 5. The first insulating substrate 1, the first reaction region forming layer 3, the first channel forming layer 4 and the first upper cover layer 5 are all made of an insulating material. The first electrode system includes a urea reference electrode 21, a urea working electrode 22, a creatinine working electrode 23, a creatinine counter electrode 24; conductive first contact 79, conductive second contact 77, conductive third contact 76 and conductive fourth contact 80 located at the first electrical connection end 7, and a first conductive trace 74 connecting the urea reference electrode 21 with the first contact 79, a second conductive trace 82 connecting the urea working electrode 22 with the second contact 77, a third conductive trace 72 connecting creatinine working electrode 23 with the third contact 76, and a fourth conductive trace 73 connecting the creatinine counter electrode 24 with the fourth contact 80. The urea reference electrode 21 and the urea working electrode 22 are located on one side of the first sample addition port 601, and the creatinine working electrode 23 and creatinine counter electrode 24 are located on the other side of the first sample addition port 601.

As shown in Figs. 1, 2, 5 and 6, the first electrode system further includes a first sample filling electrode 25 and a second sample filling electrode 26. The urea reference electrode 21, the urea working electrode 22, the creatinine working electrode 23 and the creatinine counter electrode 24 are located between the first sample filling electrode 25 and the second sample filling electrode 26, and the first sample filling electrode 25 and the second sample filling electrode 26 are connected with a seventh contact 78 and an eighth contact 75 disposed at the first electrical connection end 7 respectively by a seventh conductive trace 81 and an eighth conductive trace 71. The first sample filling electrode 25 and the second sample filling electrode 26 are made of conductive materials and may be formed on the first insulating substrate by screen printing.

The first reaction region forming layer 3 covers the first electrode system. The first reaction region forming layer 3 has three spaced reaction regions: a first reaction region 42, a second reaction region 41 and a third reaction region 43. The shapes of the reaction regions can be selected from rectangle, oval, circle, chamfered rectangle and other geometric shapes. The second reaction region 41 contains a first urea reaction reagent, the first reaction region 42 contains a second urea reaction reagent, and the third reaction region 43 contains a creatinine reaction reagent. The third reaction region 43 at least partially exposes the creatinine working electrode 23 and the creatinine counter electrode 24, the second reaction region 41 at least partially exposes the urea reference electrode 21, and the first reaction region 42 at least partially exposes the urea working electrode 22.

The first reaction region forming layer 3 also can contain a first exposure hole 44 and a second exposure hole 45. The first exposure hole 44 at least partially exposes the first sample filling electrode 25 and the second exposure hole 45 at least partially exposes the second sample filling electrode 26. The first exposure hole 44 is in communication with a first air hole 51 disposed in the first biosensor 100 on the air path, thereby facilitating flowing of the sample to the creatinine working electrode 23 and the creatinine counter electrode 24. The second exposure hole 45 is in communication with a second air hole 52 disposed in the first biosensor 100 on the air path, thereby facilitating flowing of the sample to the urea reference electrode 21 and the urea working electrode 22. After addition of the sample, whether the sample flowing to the urea reference electrode 21 and the urea working electrode 22 and the sample flowing to the creatinine working electrode 23 and the creatinine counter electrode 24 are adequate can be determined by detecting an electrical signal such as current, voltage or impedance between the first sample filling electrode 25 and the second sample filling electrode 26.

The first channel forming layer 4 is disposed on the first reaction region forming layer 3, the first channel forming layer 4 has a first channel forming area 55, a notch 62 is disposed on one side of the first channel forming layer 4, and the notch 62 is in fluid communication with the first channel forming area 55. The first channel forming area 55 is located in the middle region of the first channel forming layer 4, and the notch 62 is located in the middle part of one side of the first channel forming layer 4. The notch 62 divides the first channel forming area 55 into two parts. After a sample is added from the first sample addition port 601, the sample enters the first biosensor 100, and when the sample enters the first channel forming layer 4 through the notch 62, sample diverging occurs at this moment, with one part of the sample flowing into the first part of the first channel forming area 55 and the other part of the sample flowing into the second part of the first channel forming area 55. The sample flowing into the first part of the first channel forming area 55 eventually flows to the creatinine working electrode 23, the creatinine counter electrode 24 and the second sample filling electrode 26 through the third reaction region 43, and the sample flowing into the second part of the first channel forming area 55 eventually flows to the urea reference electrode 21, the urea working electrode 22 and the first sample filling electrode 25 through the second reaction region 41 and the first reaction region 42. The first part of the first channel forming area 55 has a width larger than that of the second part of the first channel forming area 55. The first upper cover layer 5 is disposed on the first channel forming layer 4. The first upper cover layer 5 forms a first sample channel together with the first channel forming area 55, the second reaction region 41, the first reaction region 42 and the third reaction region 43.

The first upper cover layer 5 is provided with a first air hole 51 and a second air hole 52 for discharging the air in the first sample channel during sample addition. The first air hole 51 and the second air hole 52 are in communication with the first channel forming area 55 on the air path.

The second biosensor 101 is sequentially provided with a second insulating substrate 8, a second electrode system disposed on the second insulating subsystem substrate 8, a second reaction region forming layer 10, a second channel forming layer 11 and a second upper cover layer 12. The second insulating substrate 8, the second reaction region forming layer 10, the second channel forming layer 11 and the second upper cover layer 12 are all made of an insulating material.

The second electrode system includes a uric acid counter electrode 29, a uric acid working electrode 30; conductive fifth contact 86 and conductive sixth contact 84 located at the second electrical connection end 13, a fifth conductive trace 91 connecting the uric acid counter electrode 29 with the fifth contact 86, and a sixth conductive trace 89 connecting the uric acid working electrode 30 with the sixth contact 84.

The second electrode system is further provided with a third sample filling electrode 27. The third sample filling electrode 27 is connected with a ninth contact 85 through a ninth conductive trace 90. After the sample is added, whether the sample flowing to the uric acid counter electrode 29 and the uric acid working electrode 30 is adequate can be determined by detecting the electrical signal between the third sample filling electrode 27 and the uric acid counter electrode 29 or the uric acid working electrode 30.

The second electrode system of the second biosensor is further provided with a pair of special HCT electrodes 28 for correcting the test differences caused by different HCT in the blood sample. In said pair of HCT electrodes 28, one of the HCT electrodes is connected with a tenth contact 83 through a tenth conductive trace 88, and the other HCT electrode is connected to an eleventh contact 87 through an eleventh conductive trace 92. This pair of HCT electrodes 28 is located on one side of the second sample addition port 602, and the uric acid counter electrode 29 and the uric acid working electrode 30 are located on the other side of the second sample addition port 602.

The second reaction region forming layer 10 covers the second electrode system. The second reaction region forming layer 10 has a fourth reaction region 46. The shape of the fourth reaction region 46 can be selected from rectangle, oval, circle, chamfered rectangle and other geometric shapes. The fourth reaction region 46 contains a uric acid reaction reagent. The fourth reaction region 46 partially exposes the uric acid counter electrode 29 and the uric acid working electrode 30. The second reaction region forming layer 10 has a third exposure hole 47, a fourth exposure hole 48, a fifth exposure hole 49 and a sixth exposure hole 50. The third exposure hole 47 at least partially exposes the third sample filling electrode 27. The third exposure hole 47 is in communication with the third air hole 53 disposed in the second biosensor 101 on the air path.

The fourth exposure hole 48 and the fifth exposure hole 49 each expose at least partially one of the pair of HCT electrodes 28. Among the three exposure holes, i.e. the fourth exposure hole 48, the fifth exposure hole 49 and the sixth exposure hole 50, the distance between the sixth exposure hole 50 and the second electrical connection end 13 is larger than the distance between the fifth exposure hole 49 and the second electrical connection end 13, and the distance between the fifth exposure hole 49 and the second electrical connection end 13 is larger than the distance between the fourth exposure hole 48 and the second electrical connection end 13. The sixth exposure hole 50 is located near the third exposure hole 49 to expose part of the insulating region of the second insulating substrate 8. The purpose of providing the sixth exposure hole 50 is that the sixth exposure hole 50 is in communication with the fourth air hole 54 disposed on the second biosensor 101 on the air path, thereby facilitating adequately covering the pair of HCT electrodes 28 by the sample.

The second channel forming layer 11 is disposed on the second reaction region forming layer 10, the second channel forming layer 11 has a second channel forming area 56, a notch 66 is disposed on one side of the second channel forming layer 11, and the notch 66 is in fluid communication with one end or the middle part of the second channel forming area 56. After a sample is added from the second sample addition port 602, the sample enters the second biosensor 101 and then enters the second channel forming area 56 through the notch 66 and eventually flows to the uric acid counter electrode 29 and the uric acid working electrode 30.

The notch 66 disposed on one side of the second channel forming layer 11 is in fluid communication with the middle part of the second channel forming area 56, which divides the second channel forming area 56 into two parts, so that after the sample is added from the second sample addition port 602, when the sample enters the second channel forming layer 11 through the notch 66, sample diverging occurs, with one part of the sample flowing into the first part of the second channel forming area 56 and the other part of the sample flowing into the second part of the second channel forming area 56. The sample flowing into the first part of the second channel forming area 56 eventually flows to the pair of HCT electrodes 28 through the fourth exposure hole 48 and the fifth exposure hole 49, and the sample flowing into the second part of the second channel forming area 56 eventually flows to the uric acid counter electrode 29 and the uric acid working electrode 30 through the fourth reaction region 46.

The second upper cover layer 12 is disposed on the second channel forming layer 11. The second upper cover layer 12 forms a second sample channel together with the second channel forming area 56 and the fourth reaction region 46. The second upper cover layer 12 contains a third air hole 53 and a fourth air hole 54. The second sample channel is used to provide samples to the uric acid counter electrode 29 and the uric acid working electrode 30, dissolve the reaction reagent in the fourth reaction region 46 and react to generate an electrical signal. The third air hole 53 is located above the second sample channel, and is in communication with the second sample channel on the air path to discharge the air in the second sample channel when the sample is added. The fourth air hole 54 is located above the first part of the second channel forming area 56, and is in communication with the first part of the second channel forming area 56 and the sixth exposure hole 50 on the air path to discharge the air in the second sample channel when the sample is added. The third air hole 53 is located above the second part of the second channel forming area 56, and is in communication with the second part of the second channel forming area 56 on the air path to discharge the air in the second sample channel when the sample is added.

The first upper cover layer 5, the first channel forming layer 4, the first reaction region forming area 3 and the first insulating substrate 1 are provided on the same side with a notch 61, a notch 62, a notch 63 and a notch 64, respectively. The notch 61, the notch 62, the notch 63 and the notch 64 are aligned in position. The word "align(ed)" as mentioned herein refers to that when two notches are aligned, one of the notches completely overlaps with the projected region of the other notch if the dimensions (i.e., size and/or shape) of the two notches are the same, and one of the notches is located inside the projected region of the other notch if the dimensions of the two notches are not the same. On the first biosensor 100, the notch 61, the notch 62, the notch 63 and the notch 64 form the first sample addition port 601 together. The second upper cover layer 12, the second channel forming layer 11, the second reaction region forming area 10 and the second insulating substrate 8 are provided on the same side with a notch 65, a notch 66, a notch 67 and a notch 68, respectively. The notch 65, the notch 66, the notch 67 and the notch 68 are aligned in position. On the second biosensor 101, the notch 65, the notch 66, the notch 67 and the notch 68 form the second sample addition port 602 together. When the first biosensor 100 and the second biosensor 101 are assembled together, the first sample addition port 601 and the second sample addition port 602 are located on the same side of the electrochemical test strip 200 and are aligned in position, so as to jointly form the sample addition port 6 of the electrical test strip 200.

When the first biosensor 100 and the second biosensor 101 are assembled together, a part of the sample added into the sample addition port 6 flows into the first biosensor 100 through the first sample addition port 601 and enters the first channel forming area 55 through the notch 61 and the notch 62, and the notch 62 divides the first channel forming area 55 into two parts, therefore, the sample entering the first channel forming area 55 undergoes diverging at this time, with one part of the sample flowing into the first part of the first channel forming area 55, and the other part of the sample flowing into the second part of the first channel forming area 55. The sample flowing into the first part of the first channel forming area 55 eventually flows to the creatinine working electrode 23 and the creatinine counter electrode 24 through the third reaction region 43, and the sample flowing into the second part of the first channel forming area 55 eventually flows to the urea reference electrode 21 and the urea working electrode 22 through the second reaction region 41 and the first reaction region 42. The other part of the sample added into the sample addition port 6 flows into the second biosensor 101 through the second sample addition port 602 and enters the second channel forming area 56 through the notch 65 and the notch 66, the sample entering the second channel forming area 56 undergoes diverging at this time, with one part of the sample flowing into the first part of the second channel forming area 56, and the other part of the sample flowing into the second part of the second channel forming area 56. The sample flowing into the first part of the second channel forming area 56 eventually flows to the pair of HCT electrodes 28 through the fourth exposure hole 48 and the fifth exposure hole 49 to detect the HCT value of the sample, and the sample flowing into the second part of the second channel forming area 56 eventually flows to the uric acid counter electrode 29 and the uric acid working electrode 30 through the fourth reaction region 46 to detect uric acid.

The second reaction region 41 contains a first urea reaction reagent, the first reaction region 42 contains a second urea reaction reagent, the third reaction region 43 contains a creatinine reaction reagent, and the fourth reaction region 46 contains a uric acid reaction reagent. The four reaction reagents can be respectively added to the corresponding reaction regions by methods such as solution dispensing or screen printing. The present invention combines solution dispensing and solution screen printing methods to add the four reaction reagents to the corresponding four reaction regions, respectively.

In the electrochemical test strip 200, the urea reference electrode 21 and the urea working electrode 22 detect urea in the sample by the potential measurement method, the creatinine working electrode 23 and the creatinine counter electrode 24 detect creatinine in the sample by the current measurement method, and the uric acid counter electrode 29 and the uric acid working electrode 30 detect uric acid in the sample by the current measurement method. In order to detect urea, the first urea reaction reagent contains a buffer, such as PBS; a polymer binder, such as methylcellulose; a surfactant, such as Triton X-100; and urease. The second urea reaction reagent contains a buffer, such as PBS; a polymer binder, such as methylcellulose; a surfactant, such as Triton X-100; and an electron carrier, such as a ruthenium compound (for example, hexaammineruthenium chloride). In order to detect creatinine, the creatinine reaction reagent contains a buffer, such as PBS; a polymer binder, such as methylcellulose; a stabilizer, such as sucrose; a surfactant, such as Triton X-100; creatininase, creatinekinase, and sarcosine oxidase; and an electron carrier, such as a ruthenium compound, potassium ferricyanide or potassium ferrocyanide. In order to detect uric acid, the uric acid reaction reagent contains a buffer, such as PBS; a polymer binder, such as methylcellulose; a stabilizer, such as trehalose; a surfactant, such as Triton X-100; and an electron carrier, such as a ruthenium compound, potassium ferricyanide or potassium ferrocyanide. The first urea reaction reagent and second urea reaction reagent for detecting urea and the creatinine reaction reagent for detecting creatinine can be added to the second reaction region 41, the first reaction region 42 and the third reaction region 43 respectively in a manner of solution dispensing or the like; and the uric acid reaction reagent for detecting uric acid is added to the fourth reaction region 46 through screen printing.

### Example 1 Preparation of quality control solution

The three-item quality control solution of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; 0.0001% to 3% (w/w) of sodium urate; 0.0001% to 3% (w/w) of urea; 0.0001% to 3% (w/w) of creatinine; and 0.0001% to 3% (w/w) of creatine.

The three-item quality control solution 1 of the present invention: 0.01 mol/L of TAPSO (pH 7.0); 40% (w/w) of PEG 3350; 0.02% (w/w) of calcium chloride; 1% (w/w) of EDTA; 0.1% (w/w) of sodium benzoate; 0.1% of Allura Red; 3% (w/w) of sodium urate; 0.0001% (w/w) of urea; 3% (w/w) of creatinine; and 0.0001% (w/w) of creatine.

The three-item quality control solution 2 of the present invention: 2 mol/L of TAPSO (pH 6.5); 10% (w/w) of PEG 2000; 0.5% (w/w) of calcium chloride; 0.1% (w/w) of dipotassium EDTA; 2% (w/w) of sodium benzoate; 2% of Allura Red; 0.0001% (w/w) of sodium urate; 3% (w/w) of urea; 0.0001% (w/w) of creatinine; and 3% (w/w) of creatine.

The three-item quality control solution 3 of the present invention: 1 mol/L of TAPSO (pH 7.5); 5% (w/w) of PEG6000; 0.3% (w/w) of calcium chloride; 0.1% (w/w) of disodium EDTA; 1.0% (w/w) of sodium benzoate; 0.22% of Allura Red; 0.0052% (w/w) of sodium urate; 0.2118% (w/w) of urea; 0.0066% (w/w) of creatinine; and 0.0031% (w/w) of creatine.

The three-item quality control solution 4 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0052% (w/w) of sodium urate; 0.1026% (w/w) of urea; 0.0028% (w/w) of creatinine; and 0.0020% (w/w) of creatine.

The three-item quality control solution 5 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0095% (w/w) of sodium urate; 0.1408% (w/w) of urea; 0.0044% (w/w) of creatinine; and 0.0031% (w/w) of creatine.

The three-item quality control solution 6 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0161% (w/w) of sodium urate; 0.2118% (w/w) of urea; 0.0066% (w/w) of creatinine; and 0.0046% (w/w) of creatine.

The three-item quality control solution 7 of the present invention: 0.05mol/L of HEPES (pH 6.8); 15% (w/w) of propylene glycol; 0.05% (w/w) of calcium chloride; 0.3% (w/w) of disodium EDTA; 0.15% (w/w) of sodium benzoate; 0.22% of Allura Red; 0.0096% (w/w) of sodium urate; 0.1340% (w/w) of urea; 0.0038% (w/w) of creatinine; and 0.0027% (w/w) of creatine.

The three-item quality control solution 8 of the present invention: 0.05mol/L of MES (pH 6.8); 15% (w/w) of propylene glycol; 0.05% (w/w) of calcium chloride; 0.3% (w/w) of disodium EDTA; 0.15% (w/w) of sodium benzoate; 0.22% of Allura Red; 0.0096% (w/w) of sodium urate; 0.1340% (w/w) of urea; 0.0038% (w/w) of creatinine; and 0.0027% (w/w) of creatine.

The three-item quality control solution 9 of the present invention: 0.05mol/L of BES (pH 6.8); 15% (w/w) of propylene glycol; 0.05% (w/w) of calcium chloride; 0.3% (w/w) of disodium EDTA; 0.15% (w/w) of sodium benzoate; 0.22% of Allura Red; 0.0096% (w/w) of sodium urate; 0.1340% (w/w) of urea; 0.0038% (w/w) of creatinine; and 0.0027% (w/w) of creatine.

The three-item quality control solution 10 of the present invention: 0.05mol/L of TAPSO (pH 7.0); 15% (w/w) of propylene glycol; 0.05% (w/w) of calcium chloride; 0.3% (w/w) of disodium EDTA; 0.15% (w/w) of sodium benzoate; 0.22% of Allura Red; 0.0096% (w/w) of sodium urate; 0.1340% (w/w) of urea; 0.0038% (w/w) of creatinine; and 0.0027% (w/w) of creatine.

The three-item quality control solution 11 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0059% (w/w) of sodium urate; 0.1001% (w/w) of urea; 0.0027% (w/w) of creatinine; and 0.0019% (w/w) of creatine.

The three-item quality control solution 12 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0104% (w/w) of sodium urate; 0.1525% (w/w) of urea; 0.0047% (w/w) of creatinine; and 0.0033% (w/w) of creatine.

The three-item quality control solution 13 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0160% (w/w) of sodium urate; 0.2270% (w/w) of urea; 0.0068% (w/w) of creatinine; and 0.0048% (w/w) of creatine.

The three-item quality control solution 14 of the present invention: 0.05mol/L of TAPSO (pH 7.0); 15% (w/w) of PEG 3350; 0.05% (w/w) of calcium chloride; 0.3% (w/w) of disodium EDTA; 0.15% (w/w) of sodium benzoate; 0.22% of Allura Red; 0.0087% (w/w) of sodium urate; 0.1621% (w/w) of urea; 0.0037% (w/w) of creatinine; and 0.0026% (w/w) of creatine.

The three-item quality control solution 15 of the present invention: 0.05mol/L of TAPSO (pH 7.0); 15% (w/w) of PEG 3350; 0.05% (w/w) of calcium chloride; 0.3% (w/w) of disodium EDTA; 0.15% (w/w) of sodium benzoate; 0.22% of Allura Red; 0.0087% (w/w) of sodium urate; 0.1621% (w/w) of urea; and 0.0037% (w/w) of creatinine.

The three-item quality control solution 16 of the present invention (containing neither CaCl₂ nor NaCl): 0.2mol/L of TAPSO (pH 7.0); 15% (w/w) of propylene glycol; 0.3% (w/w) of disodium EDTA; 0.15% (w/w) of sodium benzoate; 0.22% of Allura Red; 0.010% (w/w) of sodium urate; 0.1561% (w/w) of urea; 0.0058% (w/w) of creatinine; and 0.0041% (w/w) of creatine.

The three-item quality control solution 17 of the present invention (containing CaCl₂ but no NaCl): 0.2mol/L of TAPSO (pH 7.0); 15% (w/w) of propylene glycol; 0.05% (w/w) of calcium chloride; 0.3% (w/w) of disodium EDTA; 0.15% (w/w) of sodium benzoate; 0.22% of Allura Red; 0.010% (w/w) of sodium urate; 0.1561% (w/w) of urea; 0.0058% (w/w) of creatinine; and 0.0041% (w/w) of creatine.

The three-item quality control solution 18 of the present invention (containing both CaCl₂ and NaCl): 0.2mol/L of TAPSO (pH 7.0); 15% (w/w) of propylene glycol; 0.05% (w/w) of calcium chloride; 0.1% (w/w) of sodium chloride; 0.3% (w/w) of disodium EDTA; 0.15% (w/w) of sodium benzoate; 0.22% of Allura Red; 0.010% (w/w) of sodium urate; 0.1561% (w/w) of urea; 0.0058% (w/w) of creatinine; and 0.0041% (w/w) of creatine.

The two-item quality control solution a of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; 0.0001% to 3% (w/w) of sodium urate; 0.0001% to 3% (w/w) of creatinine; and 0.0001% to 3% (w/w) of creatine.

The two-item quality control solution a1 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.009% (w/w) of sodium urate; 0.0045% (w/w) of creatinine; and 0.0037% (w/w) of creatine.

The two-item quality control solution b of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; 0.0001% to 3% (w/w) of urea; 0.0001% to 3% (w/w) of creatinine; and 0.0001% to 3% (w/w) of creatine.

The two-item quality control solution b1 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.1978% (w/w) of urea; 0.0045% (w/w) of creatinine; and 0.0037% (w/w) of creatine.

The two-item quality control solution c of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; 0.0001% to 3% (w/w) of sodium urate; and 0.0001% to 3% (w/w) of urea.

The two-item quality control solution c1 of the present invention: 0.05 mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.009% (w/w) of sodium urate; and 0.1978% (w/w) of urea.

The single-item quality control solution a of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; 0.0001% to 3% (w/w) of creatinine; and 0.0001% to 3% (w/w) of creatine.

The single-item quality control solution a1 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; 0.0045% (w/w) of creatinine; and 0.0037% (w/w) of creatine.

The single-item quality control solution b of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; and 0.0001% to 3% (w/w) of urea.

The single-item quality control solution b1 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; and 0.1978% (w/w) of urea.

The single-item quality control solution c of the present invention: 0.01 mol/L to 2 mol/L of a Good's buffer with a pH of 6.5 to 7.5; 10% to 40% (w/w) of a polymer; 0.02% to 0.5% (w/w) of calcium chloride; 0.1% to 1% (w/w) of EDTA or a salt thereof; 0.1% to 2% (w/w) of a preservative; 0.1% to 2% (w/w) of a blood simulated dye; 0.0001% to 3% (w/w) of sodium urate; 0.0001% to 3% (w/w) of urea; 0.0001% to 3% (w/w) of creatinine; and 0.0001% to 3% (w/w) of creatine.

The single-item quality control solution c1 of the present invention: 0.05mol/L of TAPSO (pH 7.0); 7.5% (w/w) of PEG 3350; 7.5% (w/w) of propylene glycol; 0.05% (w/w) of calcium chloride; 0.3% (w/w) of disodium EDTA; 0.15% (w/w) of sodium benzoate; 0.22% of Allura Red; and 0.009% (w/w) of sodium urate.

In the quality control solution of the present invention, the Good's buffer can be selected from HEPES, MES, BES and TAPSO, preferably TAPSO; the blood simulated dye can be selected from Sulforhodamine B, Congo Red, Mito Red, Acid Red, Fluorescent Red, Allura Red, Cresol Red, Amaranth, Chlorophenol Red, and Sulforhodamine 101, preferably Allura Red; the polymer can be selected from PEG 2000~6000, propylene glycol, glycerol, or a combination thereof; and the preservative can be selected from sodium azide, thimerosal, sodium benzoate, Proclin-150, Proclin-200, Proclin-300 and Proclin-5000.

Preferably, a suitable amount of sodium chloride, the concentration of which is 0 to 0.1% (w/w), can also be added to the single-item quality control solution, two-item quality control solution and three-item quality control solution of the present invention.

When the quality control solutions (the single-item quality control solutions, the two-item quality control solutions and the three-item quality control solutions) of the present invention are prepared, the Good's buffer (pH 6.5 to 7.5) and the polymer are mixed at first, then EDTA or the salt thereof, the preservative and the blood simulated dye are added for mixing, and finally, at least one of sodium urate, urea and creatinine is added for adequate mixing. When the quality control solution of the present invention contains calcium chloride or sodium chloride or contains calcium chloride and sodium chloride at the same time, calcium chloride or sodium chloride should be mixed with the Good's buffer and the polymer. When the quality control solution of the preset invention contains creatine, creatine and at least one of sodium urate, urea and creatinine are added at the same time.

### Example 2: Detection of quality control solution

Before testing, the electrochemical test strip is inserted into an electrical connector of a detecting instrument, and the first and second biosensors of the electrochemical test strip are electrically connected to the electrical connector of the detecting instrument. However, after the quality control solution (the single-item quality control solution, the two-item quality control solution and the three-item quality control solution) prepared in Example 1 is added through the sample addition port for a period of time, the electrochemical signal(s) corresponding to the urea, creatinine and/or uric acid in the quality control solution is/are detected.

After addition of the quality control solution, the urea (if exists) in the quality control solution reacts with the first urea reaction reagent and the second urea reaction reagent, the urea concentration is measured using the potential method, the test time is 3s to 100s, and the detected potential signal (mV) is recorded; the creatinine (if exists) in the quality control solution reacts with the creatinine reaction reagent, the ampere current method is used for measurement, the test time is 3s to 100s, and the detected current signal (µA) is recorded; and the uric acid (if exists) in the quality control solution reacts with the uric acid reaction reagent, the ampere current method is used for measurement, the test time is 2s to 100s, and the detected current signal (µA) is recorded.

### Example 3: Evaluation of storage stability

The three-item quality control solutions 4, 5 and 6 in Example 1 are detected at room temperature (RT) using the detection method in Example 2. After placement at 65°C for 2 weeks (65C2W), the detection method in Example 2 is used to carry out the detection, and during the detection, 10 electrochemical test strips belonging to the same batch are selected for testing. The detection results are as shown in Tables 1, 2 and 3, respectively.

**Table 1. Detection results of potential signal for urea**

| urea concentration | 0.1026% (w/w) | | 0.1408% (w/w) | | 0.2118% (w/w) | |
|---|---|---|---|---|---|---|
| Stress | RT | 65C2W | RT | 65C2W | RT | 65C2W |
| Detection results | Potential value (mV) | | | | | |
| Rep. 1 | 52.69 | 54.46 | 72.19 | 74.54 | 92.46 | 94.96 |
| Rep. 2 | 52.19 | 55.38 | 70.27 | 73.73 | 94.35 | 94.73 |
| Rep. 3 | 52.15 | 54.04 | 72.65 | 74.81 | 96.04 | 92.69 |
| Rep. 4 | 53.19 | 54.31 | 72.73 | 74.12 | 92.35 | 94.31 |
| Rep. 5 | 52.73 | 54.88 | 72.35 | 73.00 | 93.69 | 90.69 |
| Rep. 6 | 51.96 | 55.50 | 71.42 | 74.23 | 94.38 | 91.54 |
| Rep. 7 | 52.12 | 54.96 | 72.58 | 73.50 | 94.19 | 93.65 |
| Rep. 8 | 53.23 | 55.96 | 71.65 | 75.96 | 92.77 | 91.50 |
| Rep. 9 | 52.15 | 55.23 | 73.12 | 75.15 | 92.23 | 92.73 |
| Rep. 10 | 53.85 | 56.15 | 72.73 | 73.92 | 91.92 | 88.62 |
| Average | 52.627 | 55.088 | 72.169 | 74.296 | 93.438 | 92.542 |
| SD | 0.63 | 0.69 | 0.84 | 0.86 | 1.31 | 2.00 |
| CV | 1.2% | 1.3% | 1.2% | 1.2% | 1.4% | 2.2% |
| %Bias vs. RT | - | 4.7% | --- | 2.9% | - | -1.0% |

**Table 2. Detection results of current signal for creatinine**

| Creatinine concentration | 0.0028% (w/w) | | 0.0044% (w/w) | | 0.0066% (w/w) | |
|---|---|---|---|---|---|---|
| Stress | RT | 65C2W | RT | 65C2W | RT | 65C2W |
| Detection results | Current value (µA) | | | | | |
| Rep. 1 | 1.27 | 1.41 | 1.75 | 1.83 | 2.18 | 2.27 |
| Rep. 2 | 1.30 | 1.35 | 1.72 | 1.87 | 2.33 | 2.29 |
| Rep. 3 | 1.22 | 1.39 | 1.79 | 1.87 | 2.23 | 2.28 |
| Rep. 4 | 1.30 | 1.33 | 1.78 | 1.91 | 2.30 | 2.32 |
| Rep. 5 | 1.32 | 1.33 | 1.77 | 1.88 | 2.24 | 2.25 |
| Rep. 6 | 1.30 | 1.36 | 1.76 | 1.87 | 2.26 | 2.21 |
| Rep. 7 | 1.26 | 1.33 | 1.69 | 1.87 | 2.34 | 2.23 |
| Rep. 8 | 1.29 | 1.29 | 1.76 | 1.89 | 2.25 | 2.31 |
| Rep. 9 | 1.30 | 1.33 | 1.75 | 1.90 | 2.21 | 2.26 |
| Rep. 10 | 1.30 | 1.38 | 1.68 | 1.86 | 2.29 | 2.29 |
| Average | 1.285 | 1.349 | 1.743 | 1.875 | 2.263 | 2.272 |
| SD | 0.03 | 0.03 | 0.04 | 0.02 | 0.05 | 0.03 |
| CV | 2.2% | 2.5% | 2.1% | 1.1% | 2.3% | 1.5% |
| %Bias vs. RT | --- | 5.0% | --- | 7.6% | --- | 0.4% |

**Table 3. Detection results of current signal for uric acid**

| Sodium urate | 0.0052% (w/w) | | 0.0095% (w/w) | | 0.0161% (w/w) | |
|---|---|---|---|---|---|---|
| Stress | RT | 65C2W | RT | 65C2W | RT | 65C2W |
| Detection results | Current value (µA) | | | | | |
| Rep. 1 | 1.54 | 1.61 | 2.37 | 2.51 | 3.84 | 3.76 |
| Rep. 2 | 1.53 | 1.60 | 2.43 | 2.38 | 3.88 | 3.82 |
| Rep. 3 | 1.52 | 1.64 | 2.43 | 2.47 | 3.99 | 3.88 |
| Rep. 4 | 1.55 | 1.62 | 2.40 | 2.40 | 3.96 | 3.99 |
| Rep. 5 | 1.57 | 1.63 | 2.43 | 2.37 | 3.96 | 3.81 |
| Rep. 6 | 1.54 | 1.61 | 2.48 | 2.43 | 3.83 | 3.87 |
| Rep. 7 | 1.54 | 1.60 | 2.41 | 2.43 | 3.84 | 3.89 |
| Rep. 8 | 1.50 | 1.61 | 2.38 | 2.44 | 3.88 | 3.98 |
| Rep. 9 | 1.55 | 1.64 | 2.47 | 2.45 | 4.04 | 3.88 |
| Rep. 10 | 1.56 | 1.64 | 2.38 | 2.47 | 3.92 | 3.94 |
| Average | 1.541 | 1.620 | 2.418 | 2.435 | 3.914 | 3.881 |
| SD | 0.02 | 0.02 | 0.04 | 0.04 | 0.07 | 0.07 |
| CV | 1.3% | 1.2% | 1.5% | 1.7% | 1.8% | 1.9% |
| %Bias vs. RT | --- | 5.2% | --- | 0.7% | --- | -0.9% |

In Table 1, at low concentration (0.1026% (w/w)), middle concentration (0.1408% (w/w)) and high concentration (0.2118% (w/w)) of urea, the potential signal for the urea is detected. In Table 2, at low concentration (0.0028% (w/w)), middle concentration (0.0044% (w/w)) and high concentration (0.0066% (w/w)) of creatinine, the current signal for the creatinine is detected. In Table 3, at low concentration (0.0052% (w/w)), middle concentration (0.0095% (w/w)) and high concentration (0.0161% (w/w)) of sodium urate, the current signal for the uric acid is detected.

Placement of the three-item quality control solutions at 65°C for 2 weeks is equivalent to storage at 30°C for 1 year. Data in Table 1, Table 2 and Table 3 indicate that the three-item quality control solutions 4, 5 and 6 placed at 65°C for 2 weeks have no particularly significant difference with the same three-item quality control solutions at room temperature in aspects of the detected potential signal for urea, current signal for creatinine and current signal for uric acid. This indicates that the three-item quality control solutions of the present invention remain stable at 30°C within 1 year.

### Example 4 Evaluation of different buffers

The three-item quality control solutions 7, 8, 9 and 10 in Example 1 are detected at room temperature (RT) using the detection method in Example 2. After placement at 65°C for 2 weeks (65C2W), and the detection method in Example 2 is used to carry out the detection. These four three-item quality control solutions differ in using different Good's buffers: HEPES (pH 6.8), MES (pH 6.8), BES (pH 6.8) and TAPSO (pH 7.0). During the detection,10 electrochemical test strips belonging to the same batch are selected for testing. The detection results are as shown in Tables 4, 5 and 6, respectively.

**Table 4. Detection results of potential signal for urea**

| Three-item quality control solution | HEPES (pH6.8) | | MES (pH6.8) | | BES (pH6.8) | | TAPSO (pH7.0) | |
|---|---|---|---|---|---|---|---|---|
| Stress | RT | 65C2W | RT | 65C2W | RT | 65C2W | RT | 65C2W |
| Detection results | Potential value (mV) | | | | | | | |
| Rep. 1 | 64.00 | 62.69 | 78.92 | 80.08 | 65.15 | 67.19 | 71.46 | 73.54 |
| Rep. 2 | 63.04 | 63.46 | 79.19 | 78.23 | 64.50 | 66.42 | 72.77 | 74.38 |
| Rep. 3 | 66.42 | 62.58 | 79.88 | 83.04 | 64.54 | 66.31 | 74.19 | 74.19 |
| Rep. 4 | 64.77 | 63.96 | 81.35 | 81.08 | 63.58 | 65.42 | 71.12 | 74.19 |
| Rep. 5 | 65.31 | 64.50 | 81.00 | 78.46 | 64.58 | 65.58 | 71.31 | 74.35 |
| Rep. 6 | 65.96 | 62.81 | 79.15 | 82.50 | 65.04 | 65.65 | 72.42 | 75.04 |
| Rep. 7 | 64.46 | 59.19 | 78.31 | 80.19 | 65.31 | 65.88 | 71.42 | 76.04 |
| Rep. 8 | 64.85 | 63.42 | 80.38 | 80.27 | 63.38 | 65.92 | 71.50 | 73.19 |
| Rep. 9 | 64.15 | 63.19 | 79.38 | 83.12 | 63.88 | 65.65 | 73.54 | 73.69 |
| Rep. 10 | 64.38 | 61.50 | 79.00 | 81.85 | 64.62 | 65.00 | 70.19 | 75.35 |
| Average | 64.735 | 62.731 | 79.658 | 80.881 | 64.458 | 65.904 | 71.992 | 74.396 |
| SD | 0.98 | 1.49 | 0.98 | 1.75 | 0.65 | 0.61 | 1.22 | 0.87 |
| CV | 1.5% | 2.4% | 1.2% | 2.2% | 1.0% | 0.9% | 1.7% | 1.2% |
| %Bias vs. RT | --- | -3.1% | --- | 1.5% | --- | 2.2% | --- | 3.3% |

**Table 5. Detection results of current signal for creatinine**

| Three-item quality control solution | HEPES (pH6.8) | | MES (pH6.8) | | BES (pH6.8) | | TAPSO (pH7.0) | |
|---|---|---|---|---|---|---|---|---|
| Stress | RT | 65C2W | RT | 65C2W | RT | 65C2W | RT | 65C2W |
| detection results | Current value (µA) | | | | | | | |
| Rep. 1 | 1.63 | 1.85 | 1.79 | 1.88 | 1.57 | 1.69 | 1.7 | 1.87 |
| Rep. 2 | 1.55 | 1.82 | 1.77 | 1.93 | 1.55 | 1.76 | 1.7 | 1.91 |
| Rep. 3 | 1.65 | 1.87 | 1.87 | 1.87 | 1.64 | 1.71 | 1.84 | 1.83 |
| Rep. 4 | 1.64 | 1.77 | 1.84 | 1.91 | 1.56 | 1.7 | 1.64 | 1.83 |
| Rep. 5 | 1.77 | 1.78 | 1.77 | 1.92 | 1.62 | 1.74 | 1.78 | 1.87 |
| Rep. 6 | 1.65 | 1.84 | 1.73 | 1.83 | 1.53 | 1.69 | 1.73 | 1.88 |
| Rep. 7 | 1.67 | 1.77 | 1.76 | 1.96 | 1.54 | 1.71 | 1.69 | 1.69 |
| Rep. 8 | 1.64 | 1.71 | 1.67 | 1.99 | 1.59 | 1.74 | 1.8 | 1.83 |
| Rep. 9 | 1.65 | 1.72 | 1.76 | 1.93 | 1.5 | 1.59 | 1.7 | 1.85 |
| Rep. 10 | 1.65 | 1.85 | 1.83 | 1.94 | 1.57 | 1.7 | 1.76 | 1.82 |
| Average | 1.649 | 1.797 | 1.779 | 1.914 | 1.567 | 1.701 | 1.732 | 1.837 |
| SD | 0.05 | 0.05 | 0.06 | 0.05 | 0.04 | 0.05 | 0.06 | 0.06 |
| CV | 3.20% | 3.00% | 3.30% | 2.40% | 2.60% | 2.70% | 3.40% | 3.20% |
| %Bias vs. RT | --- | 9.00% | --- | 7.60% | --- | 8.60% | --- | 6.00% |

**Table 6. Detection results of current signal for uric acid**

| Three-item quality control solution | HEPES (pH6.8) | | MES (pH6.8) | | BES (pH6.8) | | TAPSO (pH7.0) | |
|---|---|---|---|---|---|---|---|---|
| Stress | RT | 65C2W | RT | 65C2W | RT | 65C2W | RT | 65C2W |
| detection results | Current value (µA) | | | | | | | |
| Rep. 1 | 2.45 | 2.55 | 2.58 | 2.52 | 2.55 | 2.50 | 2.62 | 2.49 |
| Rep. 2 | 2.44 | 2.58 | 2.53 | 2.48 | 2.55 | 2.51 | 2.52 | 2.60 |
| Rep. 3 | 2.45 | 2.50 | 2.62 | 2.48 | 2.53 | 2.57 | 2.55 | 2.52 |
| Rep. 4 | 2.49 | 2.56 | 2.51 | 2.55 | 2.58 | 2.49 | 2.58 | 2.59 |
| Rep. 5 | 2.58 | 2.55 | 2.55 | 2.52 | 2.62 | 2.55 | 2.52 | 2.56 |
| Rep. 6 | 2.52 | 2.49 | 2.52 | 2.55 | 2.53 | 2.49 | 2.53 | 2.51 |
| Rep. 7 | 2.51 | 2.43 | 2.55 | 2.51 | 2.52 | 2.50 | 2.58 | 2.57 |
| Rep. 8 | 2.49 | 2.59 | 2.58 | 2.50 | 2.56 | 2.55 | 2.57 | 2.53 |
| Rep. 9 | 2.47 | 2.53 | 2.55 | 2.58 | 2.69 | 2.62 | 2.52 | 2.50 |
| Rep. 10 | 2.53 | 2.59 | 2.52 | 2.57 | 2.63 | 2.49 | 2.52 | 2.50 |
| Average | 2.494 | 2.537 | 2.551 | 2.525 | 2.575 | 2.525 | 2.552 | 2.538 |
| SD | 0.04 | 0.05 | 0.03 | 0.04 | 0.05 | 0.04 | 0.03 | 0.04 |
| CV | 1.8% | 2.0% | 1.3% | 1.4% | 2.0% | 1.7% | 1.3% | 1.6% |
| %Bias vs. RT | --- | 1.7% | --- | -1.0% | --- | -1.9% | --- | -0.5% |

It can be seen from Tables 4, 5 and 6, under these four different Good's buffers, the bias of each of the electrochemical signals for urea, creatinine and uric acid under RT and 65C2W conditions is within 10%, and the biases are relatively small. To sum up, the TAPSO (pH 7.0) buffer allows the electrochemical signals for urea, creatinine and uric acid to remain smaller biases under RT and 65C2W conditions, which is superior to the other three Good's buffers. In addition, further experiments carried out by changing the concentrations and pH of these four different Good's buffers show that when these four different Good's buffers have concentrations of 0.05mol/L to 0.5mol/L (pH 6.5 to 7.2), the electrochemical signals for urea, creatinine and uric acid can remain relatively small biases under RT and 65C2W conditions.

### Example 5: Detection precision

The three-item quality control solutions 11, 12 and 13 in Example 1 are prepared at room temperature, 3 batches of each three-item quality control solution are prepared, then each batch of the three-item quality control solutions 11, 12 and 13 are detected using the detection method in Example 2, and 10 electrochemical test strips belonging to the same batch are selected for testing before the detection. The detection results are as shown in Tables 7, 8 and 9, respectively.

**Table 7. Detection results of potential signal for urea**

| Urea concentra tion | 0.1001% (w/w) | | | 0.1525% (w/w) | | | 0.2270% (w/w) | | |
|---|---|---|---|---|---|---|---|---|---|
| Different batches of quality control solutions | 1 | 2 | 3 | 1 | 2 | 3 | 1 | 2 | 3 |
| Detection results | Potential value (mV) | | | Potential value (mV) | | | Potential value (mV) | | |
| Rep. 1 | 51.65 | 51.85 | 50.69 | 72.46 | 74.08 | 77.46 | 96.96 | 97.08 | 95.85 |
| Rep. 2 | 51.23 | 52.19 | 51.15 | 74.85 | 74.5 | 75.58 | 98.77 | 98.08 | 96.88 |
| Rep. 3 | 49.58 | 51.69 | 53.15 | 77.27 | 74.58 | 75.27 | 99.08 | 95.12 | 94.88 |
| Rep. 4 | 51.54 | 51.38 | 50.81 | 78.35 | 77.04 | 74.54 | 98.19 | 95 | 96.27 |
| Rep. 5 | 51.04 | 51.58 | 50.77 | 76.85 | 76.12 | 76.19 | 93.27 | 101.96 | 97 |
| Rep. 6 | 51.08 | 52.58 | 50.00 | 76.15 | 75.65 | 78.42 | 99.54 | 94.46 | 95.19 |
| Rep. 7 | 51.35 | 51.27 | 50.73 | 76.35 | 70.35 | 76.54 | 98.62 | 99.88 | 98.12 |
| Rep. 8 | 49.65 | 52.35 | 51.38 | 76.81 | 73.77 | 75.92 | 96.35 | 98.46 | 100.1 9 |
| Rep. 9 | 50.92 | 52.62 | 51.15 | 76 | 76.42 | 75.77 | 103 | 99.35 | 98.73 |
| Rep. 10 | 50.81 | 51.65 | 50.88 | 75.69 | 77.62 | 76.54 | 96.92 | 99.73 | 98.73 |
| Average | 50.88 5 | 51.91 6 | 51.07 1 | 76.07 8 | 75.01 3 | 76.22 3 | 98.07 | 97.912 | 97.18 4 |
| SD | 0.72 | 0.49 | 0.82 | 1.58 | 2.09 | 1.11 | 2.51 | 2.47 | 1.72 |
| CV | 1.41% | 0.94 % | 1.60 % | 2.08 % | 2.78 % | 1.45 % | 2.56 % | 2.52% | 1.77% |
| Overall | Average: 51.291, SD: 0.81, CV: 1.57% | | | Average: 75.771, SD: 1.68, CV: 2.21% | | | Average: 97.722, SD: 2.22, CV: 2.27% | | |

It can be seen from Table 7 that after addition of different batches of the three-item quality control solutions 11, 12 and 13, the CVs of the current detection results from the same batch of 10 electrochemical test strips are 1.57%, 2.21% and 2.27%, respectively, which are all smaller than 5%. This indicates that the potential values obtained from different batches of the three-item quality control solutions of the present invention have good accurate measurement precision when the same batch of electrochemical test strips are used to detect different urea concentrations.

**Table 8. Detection results of current signal for creatinine (µA) (0.0027%)**

| Creatinine concentrati on | 0.0027% (w/w) | | | 0.0047% (w/w) | | | 0.0068% (w/w) | | |
|---|---|---|---|---|---|---|---|---|---|
| Different batches of quality control solutions | 1 | 2 | 3 | 1 | 2 | 3 | 1 | 2 | 3 |
| Detection results | Current value (µA) | | | Current value (µA) | | | Current value (µA) | | |
| Rep. 1 | 1.27 | 1.27 | 1.25 | 1.9 | 1.92 | 1.86 | 2.35 | 2.43 | 2.29 |
| Rep. 2 | 1.21 | 1.31 | 1.27 | 1.85 | 1.87 | 1.81 | 2.33 | 2.33 | 2.37 |
| Rep. 3 | 1.33 | 1.28 | 1.19 | 1.81 | 1.99 | 1.88 | 2.28 | 2.25 | 2.28 |
| Rep. 4 | 1.25 | 1.32 | 1.23 | 1.85 | 1.91 | 1.79 | 2.32 | 2.32 | 2.3 |
| Rep. 5 | 1.28 | 1.24 | 1.27 | 1.8 | 1.84 | 1.9 | 2.24 | 2.32 | 2.28 |
| Rep. 6 | 1.25 | 1.22 | 1.20 | 1.8 | 1.84 | 1.85 | 2.3 | 2.27 | 2.24 |
| Rep. 7 | 1.25 | 1.23 | 1.26 | 1.87 | 1.83 | 1.87 | 2.18 | 2.25 | 2.22 |
| Rep. 8 | 1.21 | 1.28 | 1.28 | 1.85 | 1.9 | 1.8 | 2.38 | 2.31 | 2.3 |
| Rep. 9 | 1.25 | 1.26 | 1.31 | 1.85 | 1.9 | 1.71 | 2.37 | 2.28 | 2.26 |
| Rep. 10 | 1.24 | 1.26 | 1.23 | 1.85 | 1.81 | 1.86 | 2.16 | 2.32 | 2.33 |
| Average | 1.254 | 1.267 | 1.249 | 1.843 | 1.881 | 1.833 | 2.291 | 2.308 | 2.287 |
| SD | 0.03 | 0.03 | 0.04 | 0.03 | 0.05 | 0.06 | 0.08 | 0.05 | 0.04 |
| CV | 2.77% | 2.55 % | 2.96 % | 1.72 % | 2.86 % | 3.07 % | 3.33 % | 2.27% | 1.88% |
| Overall | Average: 1.257, SD: 0.03, CV: 2.74% | | | Average: 1.852, SD: 0.05, CV: 2.77% | | | Average: 2.295, SD: 0.06, CV: 2.51% | | |

It can be seen from Table 8 that after addition of different batches of the three-item quality control solutions 11, 12 and 13, the CVs of the current detection results from the same batch of 10 electrochemical test strips are 2.74%, 2.77% and 2.51%, respectively, which are all smaller than 5%. This indicates that the current values obtained from different batches of the three-item quality control solutions of the present invention have good accurate measurement precision when the same batch of electrochemical test strips are used to detect different creatinine concentrations.

**Table 9. Detection results of current signal for uric acid (µA)**

| Uric acid concentra tion | 0.0059% (w/w) | | | 0.0104% (w/w) | | | 0.0160% (w/w) | | |
|---|---|---|---|---|---|---|---|---|---|
| Different batches of quality control solutions | 1 | 2 | 3 | 1 | 2 | 3 | 1 | 2 | 3 |
| Detection results | Current value (µA) | | | Current value (µA) | | | Current value (µA) | | |
| Rep. 1 | 1.67 | 1.66 | 1.68 | 2.69 | 2.79 | 2.79 | 3.87 | 3.83 | 3.95 |
| Rep. 2 | 1.61 | 1.64 | 1.69 | 2.65 | 2.75 | 2.75 | 3.83 | 3.84 | 3.89 |
| Rep. 3 | 1.74 | 1.7 | 1.66 | 2.69 | 2.75 | 2.77 | 3.82 | 3.83 | 3.9 |
| Rep. 4 | 1.59 | 1.66 | 1.69 | 2.72 | 2.73 | 2.78 | 3.96 | 3.92 | 3.99 |
| Rep. 5 | 1.66 | 1.63 | 1.66 | 2.72 | 2.72 | 2.77 | 3.76 | 3.87 | 3.96 |
| Rep. 6 | 1.59 | 1.71 | 1.71 | 2.74 | 2.74 | 2.74 | 3.87 | 3.88 | 4.06 |
| Rep. 7 | 1.62 | 1.64 | 1.62 | 2.72 | 2.62 | 2.78 | 3.83 | 4.12 | 4.03 |
| Rep. 8 | 1.61 | 1.72 | 1.69 | 2.79 | 2.69 | 2.75 | 3.89 | 3.9 | 4.06 |
| Rep. 9 | 1.61 | 1.68 | 1.63 | 2.64 | 2.74 | 2.79 | 3.94 | 3.97 | 4.02 |
| Rep. 10 | 1.61 | 1.63 | 1.71 | 2.63 | 2.75 | 2.75 | 3.94 | 3.89 | 4.04 |
| Average | 1.631 | 1.667 | 1.674 | 2.699 | 2.728 | 2.767 | 3.871 | 3.905 | 3.99 |
| SD | 0.05 | 0.03 | 0.03 | 0.05 | 0.05 | 0.02 | 0.06 | 0.09 | 0.06 |
| CV | 2.85% | 2.02 % | 1.85 % | 1.84 % | 1.67 % | 0.66 % | 1.64 % | 2.23% | 1.57% |
| Overall | Average: 1.657, SD: 0.04, CV: 2.48% | | | Average: 2.731, SD: 0.05, CV: 1.76% | | | Average: 3.922, SD: 0.09, CV: 2.19% | | |

It can be seen from Table 9 that after addition of different batches of the three-item quality control solutions 11, 12 and 13, the CVs of the current detection results from the same batch of 10 electrochemical test strips are 2.48%, 1.76% and 2.19%, respectively, which are all smaller than 5%. This indicates that the current values obtained from different batches of the three-item quality control solutions of the present invention have good accurate measurement precision when the same batch of electrochemical test strips are used to detect different uric acid concentrations.

### Example 6: Evaluation of influence of creatine

The three-item quality control solutions 14 and 15 in Example 1 are prepared at room temperature (RT). The three-item quality control solution 14 differs from the three-item quality control solution 15 in that creatine is added to the former but no creatine is added to the later. After conversion, the three-item quality control solutions 14 and 15 have a uric acid concentration of about 460 µmol/L, a creatinine concentration of about 330 µmol/L, and a urea concentration of about 27 mmol/L. Mindray BS-350E fully automated biochemical analyzer is used to detect the uric acid, urea and creatinine concentrations (µmol/L) of the three-item quality control solutions 14 and 15 at room temperature, and the detection is repeated 2 times; and after storage of the three-item quality control solutions 14 and 15 at 65°C for 2 weeks, this biochemical analyzer is used again to detect the uric acid, urea and creatinine concentrations (µmol/L) of the three-item quality control solutions 14 and 15, and the detection is repeated 2 times. The detection results are as shown in Table 10.

**Table 10. Evaluation of influence of creatine**

| | Three-item quality control solution 14 | | Three-item quality control solution 15 | |
|---|---|---|---|---|
| Stress | RT | 65C2W | RT | 65C2W |
| Detection object | Creatinine (µmol/L) | | | |
| Rep. 1 | 353.3 | 365.3 | 355.6 | 300.8 |
| Rep. 2 | 351.3 | 368.3 | 351.7 | 299.5 |
| Average | 352.30 | 366.80 | 353.65 | 300.15 |
| | 6.76% | 11.2% | 7.17% | |
| %Bias vs. RT | --- | 4.1% | --- | -15.1% |

| Detection object | Urea (mmol/L) | | | |
|---|---|---|---|---|
| Rep. 1 | 23.98 | 23.97 | 23.37 | 23.21 |
| Rep. 2 | 24.30 | 23.66 | 23.44 | 23.39 |
| Average | 24.140 | 23.815 | 23.405 | 23.300 |
| %Bias vs. RT | --- | -1.3% | --- | -0.4% |

| Detection object | Uric acid (µmol/L) | | | |
|---|---|---|---|---|
| UA 1 | 502 | 468 | 509 | 488 |
| UA 2 | 496 | 464 | 510 | 487 |
| Average | 499.0 | 466.0 | 509.5 | 487.5 |
| %Bias vs. RT | --- | -6.6% | --- | -4.3% |

It can be seen from Table 10 that in the case of co-presence of creatinine and creatine, after storage at 65°C for 2 weeks, the biochemical test value of the creatinine in the three-item quality control solution 14 has no significant change, but the biochemical test value of the creatinine in the three-item quality control solution 15 decreases 15%, and the content of the creatinine changes significantly. The presence of creatine can effectively keep the content of the creatinine in the three-item quality control solution 14 stable.

### Example 7: Evaluation of influence of sodium chloride and calcium chloride

The three-item quality control solutions 16, 17 and 18 prepared at room temperature (RT) in Example 1 are detected using the detection method in Example 2. At the same time, said prepared three-item quality control solutions 16, 17 and 18 are placed at 65°C for 2 weeks (65C2W), then the detection method in Example 2 is used to carry out the detection, and the same batch of 5 electrochemical test strips are selected for testing before the detection. The detection results are as shown in Tables 11, 12 and 13, respectively.

**Table 11**

| | Three-item quality control solution 16 | | | | | |
|---|---|---|---|---|---|---|
| Stress | RT | 65C2W | RT | 65C2W | RT | 65C2W |
| Detection results | Current value for creatinine (µA) | | Potential value for urea (mV) | | Current value for uric acid (µA) | |
| Rep. 1 | 1.67 | 1.67 | 66.46 | 66.62 | 2.99 | 2.73 |
| Rep. 2 | 1.62 | 1.78 | 61.00 | 63.12 | 2.83 | 2.83 |
| Rep. 3 | 1.62 | 1.71 | 61.04 | 62.15 | 2.95 | 2.71 |
| Rep. 4 | 1.60 | 1.74 | 59.04 | 63.85 | 2.80 | 2.97 |
| Rep. 5 | 1.55 | 1.78 | 63.58 | 67.65 | 2.79 | 2.88 |
| Average | 1.609 | 1.736 | 62.223 | 64.677 | 2.871 | 2.825 |
| SD | 0.04 | 0.05 | 2.87 | 2.35 | 0.09 | 0.11 |
| CV | 2.6% | 2.7% | 4.6% | 3.6% | 3.1% | 3.8% |
| %Bias vs. RT | --- | 7.9% | --- | 3.9% | --- | -1.6% |

**Table 12**

| | Three-item quality control solution 17 | | | | | |
|---|---|---|---|---|---|---|
| Stress | RT | 65C2W | RT | 65C2W | RT | 65C2W |
| Detection results | Current value for creatinine (µA) | | Potential value for urea (mV) | | Current value for uric acid (µA) | |
| Rep. 1 | 1.66 | 1.80 | 71.46 | 71.50 | 2.78 | 2.69 |
| Rep. 2 | 1.68 | 1.76 | 68.15 | 68.04 | 2.76 | 2.71 |
| Rep. 3 | 1.70 | 1.78 | 68.96 | 67.73 | 2.82 | 2.72 |
| Rep. 4 | 1.65 | 1.84 | 64.19 | 68.27 | 2.81 | 2.68 |
| Rep. 5 | 1.67 | 1.75 | 66.27 | 70.58 | 2.82 | 2.67 |
| Average | 1.671 | 1.787 | 67.808 | 69.223 | 2.797 | 2.694 |
| SD | 0.02 | 0.04 | 2.75 | 1.70 | 0.03 | 0.02 |
| CV | 1.1% | 2.1% | 4.1% | 2.5% | 0.9% | 0.8% |
| %Bias vs. RT | --- | 6.9% | --- | 2.1% | --- | -3.7% |

**Table 13**

| | Three-item quality control solution 18 | | | | | |
|---|---|---|---|---|---|---|
| Stress | RT | 65C2W | RT | 65C2W | RT | 65C2W |
| Detection results | Current value for creatinine (µA) | | Potential value for urea (mV) | | Current value for uric acid (µA) | |
| Rep. 1 | 1.89 | 1.92 | 59.08 | 58.35 | 2.88 | 2.89 |
| Rep. 2 | 1.89 | 1.91 | 57.23 | 59.54 | 3.02 | 2.70 |
| Rep. 3 | 1.86 | 1.93 | 64.15 | 64.31 | 2.93 | 2.87 |
| Rep. 4 | 1.87 | 1.88 | 56.12 | 58.92 | 2.96 | 2.81 |
| Rep. 5 | 1.85 | 1.91 | 59.35 | 57.04 | 2.94 | 2.88 |
| Average | 1.872 | 1.910 | 59.185 | 59.631 | 2.945 | 2.831 |
| SD | 0.02 | 0.02 | 3.08 | 2.77 | 0.05 | 0.08 |
| CV | 0.9% | 1.0% | 5.2% | 4.6% | 1.8% | 2.8% |
| %Bias vs. RT | --- | 2.0% | --- | 0.8% | --- | -3.9% |

It can be seen from Tables 11, 12 and 13, the bias of each of the electrochemical signals for creatinine, urea and uric acid in the three-item quality control solutions 16, 17 and 18 under RT and 65C2W conditions is within 10%, and the biases are relatively small. This indicates that the three-item quality control solutions 16, 17 and 18 of the present invention remain stable at 30°C within 1 year. In addition, in the three-item quality control solution 18, in the presence of CaCl₂ and NaCl, the bias of each of the electrochemical signals for creatinine, urea and uric acid is smaller than 4% under RT and 65C2W conditions, while in the three-item quality control solution 16, the bias of the electrochemical signal for creatinine is 7.9% under RT and 65C2W conditions, which is smaller than 10% but greater than 5%; and in the three-item quality control solution 17, the bias of the electrochemical signal for creatinine is 6.9% under RT and 65C2W conditions, which is smaller than 10% but greater than 5%. In addition, it also can be seen from Tables 11, 12 and 13 that a suitable amount of NaCl is added, which can serve the function of enhancing the ion strength, increase test current values of uric acid and creatinine and improve electrical signal gradients.

**Example 8: Evaluation of storage stability of other quality control solutions of the**

### present invention

The single-item quality control solutions a1, b1 and c1 and two-item quality control solutions a1, b1 and c1 in Example 1 are detected at room temperature using the detection method in Example 2, then after placement at room temperature (RT) and 65°C for 2 weeks (65C2W), the detection method in Example 2 is used to carry out the detection, and during the detection, the same batch of 10 electrochemical test strips are selected for testing. The detection results are as shown in Tables 14, 15, 16 and 17, respectively.

**Table 14**

| | Single-item quality control solution a1 | | Single-item quality control solution b1 | | Single-item quality control solution c1 | |
|---|---|---|---|---|---|---|
| Stress | RT | 65C2W | RT | 65C2W | RT | 65C2W |
| Detection results | Current value for creatinine (µA) | | Potential value for urea (mV) | | Current value for uric acid (µA) | |
| Rep. 1 | 1.98 | 1.83 | 88.15 | 86.85 | 2.50 | 2.53 |
| Rep. 2 | 2.01 | 1.75 | 88.96 | 87.65 | 2.45 | 2.59 |
| Rep. 3 | 1.94 | 1.79 | 88.46 | 86.46 | 2.47 | 2.54 |
| Rep. 4 | 1.95 | 1.81 | 88.46 | 86.46 | 2.42 | 2.49 |
| Rep. 5 | 1.84 | 1.83 | 86.77 | 89.12 | 2.49 | 2.48 |
| Rep. 6 | 1.97 | 1.78 | 86.96 | 87.92 | 2.45 | 2.67 |
| Rep. 7 | 2.04 | 1.80 | 88.35 | 86.58 | 2.45 | 2.49 |
| Rep. 8 | 1.98 | 1.75 | 87.73 | 86.19 | 2.49 | 2.58 |
| Rep. 9 | 1.88 | 1.71 | 87.12 | 86.96 | 2.44 | 2.49 |
| Rep. 10 | 1.90 | 1.74 | 89.08 | 86.04 | 2.43 | 2.56 |
| Average | 1.948 | 1.778 | 88.004 | 87.023 | 2.458 | 2.543 |
| SD | 0.06 | 0.04 | 0.82 | 0.95 | 0.03 | 0.06 |
| CV | 3.1% | 2.3% | 0.9% | 1.1% | 1.1% | 2.3% |
| %Bias vs. RT | --- | -8.7% | --- | -1.1% | --- | 3.4% |

It can be seen from Table 14, the bias of each of the electrochemical signal for creatinine in the single-item quality control solution a1, the electrochemical signal for urea in the single-item quality control solution b1, and the electrochemical signal for uric acid in the single-item quality control solution c1 under RT and 65C2W conditions is within 10%, and the biases are relatively small. This indicates that the single-item quality control solutions a1, b1 and c1 of the present invention remain stable at 30°C within 1 year.

**Table 15**

| | Two-item quality control solution a1 | | | |
|---|---|---|---|---|
| Stress | RT | 65C2W | RT | 65C2W |
| Detection results | Current value for creatinine (µA) | | Potential value for uric acid (µA) | |
| Rep. 1 | 1.90 | 1.98 | 2.45 | 2.48 |
| Rep. 2 | 2.00 | 1.94 | 2.50 | 2.43 |
| Rep. 3 | 1.91 | 1.97 | 2.57 | 2.51 |
| Rep. 4 | 1.72 | 1.97 | 2.52 | 2.51 |
| Rep. 5 | 1.89 | 2.04 | 2.45 | 2.43 |
| Rep. 6 | 2.04 | 1.85 | 2.48 | 2.43 |
| Rep. 7 | 2.01 | 1.84 | 2.52 | 2.45 |
| Rep. 8 | 1.92 | 1.80 | 2.49 | 2.41 |
| Rep. 9 | 1.91 | 1.99 | 2.49 | 2.49 |
| Rep. 10 | 1.93 | 1.98 | 2.55 | 2.47 |
| Average | 1.922 | 1.934 | 2.502 | 2.461 |
| SD | 0.09 | 0.08 | 0.04 | 0.04 |
| CV | 4.5% | 4.1% | 1.5% | 1.5% |
| %Bias vs. RT | --- | 0.6% | --- | -1.7% |

**Table 16**

| | Two-item quality control solution b1 | | | |
|---|---|---|---|---|
| Stress | RT | 65C2W | RT | 65C2W |
| Detection results | Current value for creatinine (µA) | | Potential value for urea (mV) | |
| Rep. 1 | 1.99 | 2.17 | 87.04 | 93.04 |
| Rep. 2 | 1.87 | 2.16 | 90.46 | 90.77 |
| Rep. 3 | 1.97 | 2.14 | 88.38 | 91.23 |
| Rep. 4 | 1.93 | 2.12 | 88.46 | 94.15 |
| Rep. 5 | 1.96 | 1.99 | 89.12 | 91.85 |
| Rep. 6 | 1.85 | 2.03 | 87.08 | 89.19 |
| Rep. 7 | 2.01 | 2.14 | 88.00 | 96.27 |
| Rep. 8 | 1.87 | 2.11 | 87.69 | 95.88 |
| Rep. 9 | 1.98 | 2.11 | 88.62 | 90.85 |
| Rep. 10 | 1.94 | 2.04 | 88.42 | 95.85 |
| Average | 1.935 | 2.099 | 88.327 | 92.908 |
| SD | 0.05 | 0.06 | 1.00 | 2.52 |
| CV | 2.8% | 2.8% | 1.1% | 2.7% |
| %Bias vs. RT | --- | 8.5% | --- | 5.2% |

**Table 17**

| | Two-item quality control solution c1 | | | |
|---|---|---|---|---|
| Stess | RT | 65C2W | RT | 65C2W |
| Detection results | Current value for uric acid (µA) | | Potential value for urea (mV) | |
| Rep. 1 | 2.50 | 2.57 | 88.35 | 91.00 |
| Rep. 2 | 2.50 | 2.56 | 89.27 | 91.50 |
| Rep. 3 | 2.47 | 2.59 | 87.77 | 92.46 |
| Rep. 4 | 2.87 | 2.57 | 89.46 | 91.31 |
| Rep. 5 | 2.47 | 2.54 | 87.73 | 91.15 |
| Rep. 6 | 2.49 | 2.58 | 88.00 | 90.69 |
| Rep. 7 | 2.49 | 2.57 | 86.58 | 91.19 |
| Rep. 8 | 2.41 | 2.53 | 87.65 | 91.42 |
| Rep. 9 | 2.50 | 2.52 | 86.88 | 89.42 |
| Rep. 10 | 2.52 | 2.59 | 90.42 | 92.35 |
| Average | 2.521 | 2.561 | 88.212 | 91.250 |
| SD | 0.13 | 0.02 | 1.19 | 0.85 |
| CV | 5.0% | 0.9% | 1.4% | 0.9% |
| %Bias vs. RT | --- | 1.6% | --- | 3.4% |

It can be seen from Tables 15, 16 and 17, the bias of each of the electrochemical signals for creatinine and uric acid in the two-item quality control solution a1, the electrochemical signals for creatinine and urea in the two-item quality control solution b1, and the electrochemical signals for uric acid and urea in the two-item quality control solution c1 under RT and 65C2W conditions is within 10%, and the biases are relatively small. This indicates that the two-item quality control solutions a1, b1 and c1 of the present invention remain stable at 30°C within 1 year.

## Claims

1. A quality control solution comprises a buffer, a polymer, EDTA or a salt thereof, a preservative, a blood simulated dye and an analyte.

2. The quality control solution of claim 1, wherein the buffer has a concentration of 0.01 mol/L to 2 mol/L and a pH of 6.5 to 7.5, the concentration of the polymer is 10% to 40% (w/w), the concentration of EDTA or the salt thereof is 0.1% to 1% (w/w), the concentration of the preservative is 0.1% to 2% (w/w), and the concentration of the blood simulated dye is 0.1% to 2% (w/w).

3. The quality control solution of claim 1, wherein said analyte is selected from urea, sodium urate, creatinine or a combination thereof.

4. The quality control solution of claim 3, wherein when said analyte comprises creatinine, the concentration of the creatinine is 0.0001% to 3% (w/w); when said analyte comprises sodium urate, the concentration of the sodium urate is 0.0001% to 3% (w/w); and when said analyte comprises urea, the concentration of the urea is 0.0001% to 3% (w/w).

5. The quality control solution of claim 1, wherein said polymer is selected from PEG 2000~6000, propylene glycol, glycerol, or a combination thereof.

6. The quality control solution of claim 1, wherein said quality control solution further comprises calcium chloride or sodium chloride, or a combination of calcium chloride and sodium chloride.

7. The quality control solution of claim 6, wherein when said quality control solution comprises calcium chloride, the concentration of the calcium chloride is 0.02% to 0.5% (w/w); and when said quality control solution comprises sodium chloride, the concentration of the sodium chloride is 0 to 0.1% (w/w).

8. The quality control solution as claimed in claim 2, wherein when said analyte comprises creatinine, said quality control solution further comprises creatine, and preferably, the creatine has a concentration of 0.0001% to 3% (w/w).

9. The quality control solution of claim 1, wherein said buffer is a Good's buffer selected from HEPES, MES, BES and TAPSO, and preferably, said Good's buffer has a concentration of 0.05 mol/L to 0.5 mol/L and a pH of 6.5 to 7.2.

10. Use of the quality control solution of one of claims 1 to 9 in detection of urea, creatinine and/or uric acid.
